# EUROPÄISCHE PATENTSCHRIFT

(11) **EP 4 563 115 B1**
(45) Veröffentlichungstag und Bekanntmachung des Hinweises auf die Patenterteilung: **02.09.2026**
(21) Anmeldenummer: 23213076.5
(22) Anmeldetag: 29.11.2023
(51) Int. Cl.: A61C 13/00, A61C 13/08, A61C 13/083, B33Y 10/00, B33Y 40/20, B33Y 80/00, A61K 6/802

(54) **FIXIERUNG VON FÄRBELÖSUNGEN BEIM KERAMISCHEN MULTI-COLOR-INKJET-3D-DRUCK**
FIXATION OF DYE SOLUTIONS IN MULTI-COLOR INK JET 3D CERAMIC PRINTING
FIXATION DE SOLUTIONS DE COLORANT DANS L'IMPRESSION 3D PAR JET D'ENCRE EN CÉRAMIQUE MULTICOLORE

(43) Veröffentlichungstag der Anmeldung: 04.06.2025
(73) Patentinhaber: Ivoclar Vivadent AG, 9494 Schaan (LI)
(72) Erfinder: John, Hendrik, 9470 Buchs (CH); Rothbrust, Frank, 6822 Röns (AT); Krolikowski, Sebastian, 8805 Richterswil (CH); Ritzberger, Christian, 9472 Grabs (CH)
(74) Vertreter: Baldus, Oliver

(56) Entgegenhaltungen:
- EP-A1- 2 529 694
- DE-U1- 202014 101 612

## Beschreibung

Die vorliegende Erfindung betrifft ein Verfahren zum Herstellen einer dentalen Restauration durch Strahldruck und ein Herstellungsgerät zum Herstellen einer dentalen Restauration durch Strahldruck.

Es wird in diesem Zusammenhang die EP 2 529 694 A1 genannt, welche ein solches Verfahren und Herstellungsgerät offenbart.

Bei der manuellen Einfärbung von Keramik-Restaurationen aus Zirkondioxid werden zur individuellen Einfärbung Infiltrationslösungen aus Nitratsalzen eingesetzt. Diese werden beispielsweise mittels eines Pinsels auf die Oberfläche der Restauration aufgetragen und diffundieren anschliessend in die poröse Keramik. Dieses Auftragen erfordert ein hohes Maß an Erfahrung des Zahntechnikers.

Dieser Einfärbeprozess von Zirkondioxid-Weißlingen kann automatisiert werden, indem ein Airbrush-System die Restauration computergesteuert infiltriert. Bei additiven Fertigungsverfahren können verschieden eingefärbte, lösungsmittel-basierte keramische Schlicker selektiv mittels Inkjet aufgetragen werden. Die Verarbeitung von hochgefüllten, keramischen Schlickern stellt hohe Anforderungen an die eingesetzten Druckköpfe in Bezug auf eine Partikelgröße, einen Füllgrad und somit eine Viskosität und Abrasion. Das Strahldrucken (Jetten) von wässrigen Schlickern stellt eine Alternative zu den lösungsmittel-basierten Schlickern dar. Dabei wird die gejettete Materialschicht rissfrei getrocknet.

Bei einem manuellen Infiltrationsvorgang diffundieren die Salzlösungen jedoch unkontrolliert in den Zirkondioxid-Weißling. Bei einem schichtweisen Materialauftrag in Kombination mit einem schichtweisen, selektiven Einfärben der zuvor aufgetragenen Keramikschicht, werden die zuvor deponierten Nitratsalze in den unterliegenden Schichten wieder gelöst und diffundieren unkontrolliert weiter in den bereits gefertigten Keramikkörper.

Es ist die technische Aufgabe der vorliegenden Erfindung, das unkontrollierte Diffundieren oder Verlaufen von selektiv aufgetragenen Nitratsalzlösungen als Färbelösungen während eines räumlichen Schichtaufbauverfahrens zu verhindern.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration durch Strahldruck gelöst, mit den Schritten eines Strahldruckens einer oder mehrerer Schichten der dentalen Restauration mittels eines keramischen Schlickers; eines Strahldruckens einer Färbelösung auf die eine oder mehreren Schichten; und eines Auftragens einer Fixierlösung zum Fixieren der Färbelösung. Dadurch kann das unkontrollierte Diffundieren oder Verlaufen von selektiv aufgetragenen Nitratsalzlösungen als Färbelösung während eines Schichtaufbauverfahrens unterbunden werden. Es wird eine kontrollierte, selektive Einfärbung des 3D-gedruckten Grünlings mittels Färbelösungen während des additiven Herstellverfahrens gewährleistet. Ein selektiver Materialauftrag des Schlickers und eine selektive Einfärbung erfolgen dabei getrennt. Für den Materialauftrag und die Einfärbung können die gleichen oder unterschiedliche Druckköpfe eingesetzt werden. Durch die Fixierlösung wird beispielsweise die Diffusion der Färbelösung auf dem wässrigen Schlicker zur Herstellung eines Multicolor-Grünlings verhindert. Auf diese Weise wird beispielsweise eine kontrollierte, selektive Einfärbung des 3D-gedruckten ZrO2-Grünlings mittels Färbelösungen während des additiven Herstellverfahrens gewährleistet.

In einer technisch vorteilhaften Ausführungsform des Verfahrens wird die Fixierlösung auf die eine oder mehreren Schichten gesprüht. Dadurch wird beispielsweise der technische Vorteil erreicht, dass die Fixierlösung großflächig und schnell aufgetragen werden kann.

In einer weiteren technisch vorteilhaften Ausführungsform des Verfahrens wird die Fixierlösung auf die eine oder mehreren Schichten mittels eines Druckkopfes strahlgedruckt. Dadurch wird beispielsweise der technische Vorteil erreicht, dass die Fixierlösung zielgenau selektiv aufgetragen werden kann.

In einer weiteren technisch vorteilhaften Ausführungsform des Verfahrens werden die eine oder mehreren Schichten vor oder nach dem Strahldrucken der Färbelösung getrocknet. Dadurch wird beispielsweise der technische Vorteil erreicht, dass ein Diffundieren der Färbelösung verringert wird.

In einer weiteren technisch vorteilhaften Ausführungsform des Verfahrens werden die eine oder mehreren Schichten vor oder nach dem Auftragen der Fixierlösung getrocknet. Dadurch wird beispielsweise ebenfalls der technische Vorteil erreicht, dass ein Diffundieren der Färbelösung verringert wird.

In einer weiteren technisch vorteilhaften Ausführungsform des Verfahrens ist ein pH-Wert der Fixierlösung größer als 7.

Dadurch wird beispielsweise der technische Vorteil erreicht, dass ein Verlaufen der Färbelösung wirksam verringert wird.

In einer weiteren technisch vorteilhaften Ausführungsform des Verfahrens ist die Fixierlösung eine Ammoniak-AmmoniumchloridLösung. Dadurch wird beispielsweise der technische Vorteil erreicht, dass die Färbelösungen lokal fixiert werden.

In einer weiteren technisch vorteilhaften Ausführungsform des Verfahrens werden die Schritte zum Herstellen der dentalen Restauration wiederholt. Dadurch wird beispielsweise der technische Vorteil erreicht, dass die gesamte dentale Restauration aufgebaut und eingefärbt werden kann.

In einer weiteren technisch vorteilhaften Ausführungsform des Verfahrens wird die dentale Restauration in einem Sinterofen gesintert. Dadurch wird beispielsweise der technische Vorteil erreicht, dass eine dentale Restauration mit hoher Festigkeit hergestellt werden kann.

Gemäß einem zweiten Aspekt wird die technische Aufgabe durch ein Herstellungsgerät zum Herstellen einer dentalen Restauration durch Strahldruck gelöst, mit einem ersten Druckkopf zum Strahldrucken einer oder mehrerer Schichten der dentalen Restauration mittels eines keramischen Schlickers; und einem zweiten Druckkopf zum Strahldrucken einer Färbelösung auf die eine oder mehreren Schichten; und einer Auftragungsvorrichtung zum Auftragen einer Fixierlösung zum Fixieren der Färbelösung. Durch das Herstellungsgerät werden die gleichen technischen Vorteile wie durch das Verfahren nach dem ersten Aspekt erreicht.

In einer technisch vorteilhaften Ausführungsform des Herstellungsgeräts ist die Auftragungsvorrichtung durch einen Sprühkopf zum flächigen Auftragen der Fixierlösung gebildet.

Dadurch wird beispielsweise der technische Vorteil erreicht, dass die Fixierlösung großflächig und schnell aufgetragen werden kann.

In einer weiteren technisch vorteilhaften Ausführungsform des Herstellungsgeräts ist die Auftragungsvorrichtung durch einen Druckkopf zum selektiven Auftragen der Fixierlösung gebildet. Dadurch wird beispielsweise der technische Vorteil erreicht, dass die Fixierlösung zielgenau selektiv aufgetragen werden kann.

Erfindungsgemäß umfasst das Herstellungsgerät einen Aufnahmebehälter mit einer Fixierlösung. Der Aufnahmebehälter kann auswechselbar sein. Dadurch wird beispielsweise der technische Vorteil erreicht, dass die Fixierlösung vorgehalten und auf einfache Weise ersetzt werden kann.

In einer weiteren technisch vorteilhaften Ausführungsform des Herstellungsgeräts umfasst das Herstellungsgerät eine Trocknungsvorrichtung zum Trocknen der aufgetragenen Materialien, wie beispielsweise eine Schlickerschicht, Färbelösung, Fixierlösung. Dadurch wird beispielsweise der technische Vorteil erreicht, dass ein weiteres Diffundieren vermindert werden kann.

In einer weiteren technisch vorteilhaften Ausführungsform des Herstellungsgeräts umfasst die Trocknungsvorrichtung ein Gebläse und/oder einen Infrarotstrahler. Dadurch wird beispielsweise der technische Vorteil erreicht, dass die Schichten schnell getrocknet werden können.

Ausführungsbeispiele der Erfindung sind in den Zeichnungen dargestellt und werden im Folgenden näher beschrieben.

Es zeigen:
- Fig. 1: eine Darstellung unterschiedlicher Bereiche eines Schneidezahns;
- Fig. 2: eine schematische Darstellung einer dentalen Restauration;
- Fig. 3: eine schematische Darstellung eines Herstellungsgeräts zum Herstellen einer dentalen Restauration;
- Fig. 4: eine schematische Ansicht eines Verfahrens zum Herstellen einer dentalen Restauration durch Strahldruck;
- Fig. 5: eine schematische Ansicht eines weiteren Verfahrens zum Herstellen einer dentalen Restauration durch Strahldruck;
- Fig. 6: eine Tabelle mit den Zusammensetzungen unterschiedlicher Färbelösungen; und
- Fig. 7: ein Blockdiagramm eines Verfahrens zum Herstellen einer dentalen Restauration.

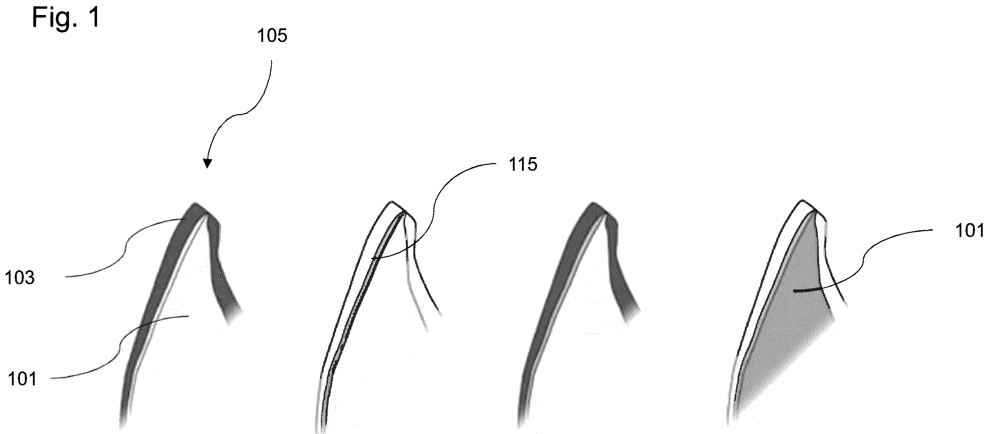

Fig. 1 zeigt eine Darstellung unterschiedlicher Bereiche eines Zahns 105. Der Zahn 105 umfasst einen inneren Dentinkern 101 und einen äußeren Zahnschmelz 103. Für die Grundeinfärbung des Zahns 105 ist der opake, d.h. undurchsichtige Dentinkern 101 verantwortlich. Dieser scheint durch den Zahnschmelz 103 der Schneide hindurch. Der Zahnschmelz 103 ist naturgemäß durchscheinend, d.h. transluzent. Transluzenz ist die partielle Lichtdurchlässigkeit eines Körpers. Um eine dentale Restauration möglichst naturgetreu aussehen zu lassen, wird dieser Aufbau des Zahns 105 auch in künstlichen dentalen Restaurationen verwendet. Hierzu werden Materialien mit unterschiedlichen optischen Eigenschaften bei der Herstellung der dentalen Restauration verwendet.

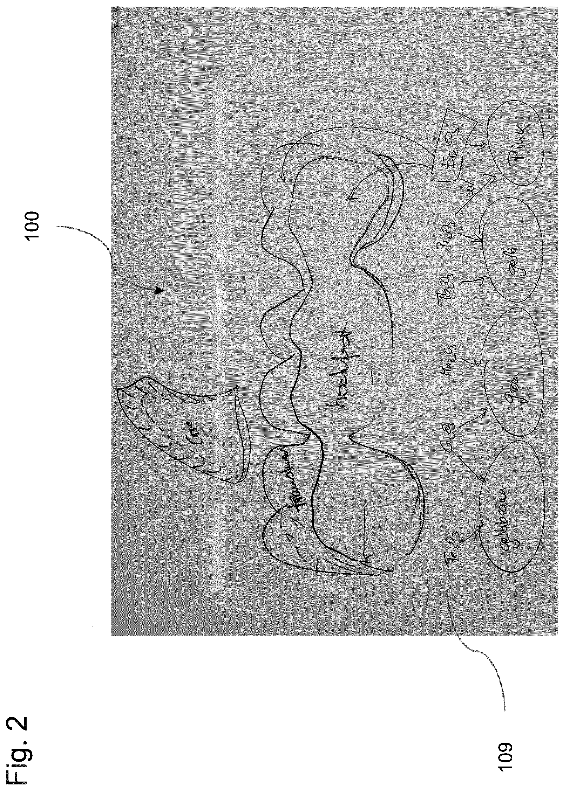

Fig. 2 zeigt eine schematische Darstellung einer dentalen Restauration 100. Die dentale Restauration 100 dient als Zahnersatz und ist beispielsweise durch eine Brücke, Krone, Voll- oder Teilprothese gebildet. Die dentale Restauration 100 wird beispielsweise aus 1-n unterschiedlichen keramischen Schlickern 109 mittels eines Strahldruckverfahrens schichtweise selektiv aufgebaut und erhält nach einer schichtweisen selektiven Einfärbung mittels Nitratsalzen wie z.B. Fe2O3, Cr2O3, Mn2O3, Tb2O3, Pr2O3, Er2O3, Co3O4, NiO, TiO2, CeO2 und/oder Dotierung mittels **z.B.** Yttrium und einer abschließenden Sinterung des so erstellten Weisslings der dentalen Restauration 100 eine entsprechende Charakterisierung in Bezug auf Farbe, Transluzenz, Opazität und mechanischer Eigenschaften.

Die dentale Restauration 100 wird dabei durch aufeinanderfolgende Schichten aufgebaut, die aufeinander gedruckt werden. Dabei können die Keramikpulver der Schlicker 109 bereits in vorgegebenen Transluzenzen bereitgestellt sein. Durch die Mischung dieser Schlicker 109 ergibt sich die Soll-Transluzenz der dentalen Restauration 100 in dem jeweiligen räumlichen Bereich.

Nach dem selektiven Materialauftrag einer Schicht des Schlickers 109 mittels des Strahldruckverfahrens wird diese Schicht rissfrei getrocknet, indem das Wasser oder das Lösungsmittel als Binder verdampft wird. Zurück bleibt eine poröse Weißling-Schicht, die eine Schichtdicke von 1 um bis 50 um und eine Dichte von mindestens 2.5 g/cm³ aufweist. Dieser Prozess wird wiederholt, bis die gesamte dentale Restauration 100 schichtweise räumlich aufgebaut ist.

Im Falle von ZrO2-Schlickern 109 können für die verschiedenen Festigkeiten auch unterschiedliche Yttrium-dotierte Keramikpulver (3 mol% Yttrium - 3Y-TZP, 4 mol% Yttrium - 4Y-TZP, 5 mol% Yttrium - 5Y-TZP) zum Einsatz kommen. Dabei ist die Yttrium-Dotierung auch für den Grad der Transluzenz ursächlich. Die unterschiedlichen Yttrium-dotierten Keramikpulver weisen unterschiedliche Eigenschaften auf.
3Y-TZP = niedrige Transluzenz / hohe Festigkeit
4Y-TZP = mittlere Transluzenz / mittlere Festigkeit
5Y-TZP = hohe Transluzenz / niedrige Festigkeit

Werden jedoch unterschiedlich voreingefärbte Schlicker 109 verwendet, die alle auch eine unterschiedliche Transluzenz und/oder mechanische Eigenschaften aufweisen, ist jedem dieser Schlicker 109 ein eigener Druckkopf 111-1 zugeordnet. Wird beispielsweise ein allgemeines CYMK-Farbschema eingesetzt, um den gesamten Farbraum abzudecken, sind 3 x 4 = 12 Schlicker 109 in 12 Druckköpfen enthalten. Auch die Herstellung dieser verschiedenen Schlicker 109 ist aufwendig. Diese große Anzahl an Schlickern 109 werden als unterschiedliche Artikel vorgehalten und gepflegt.

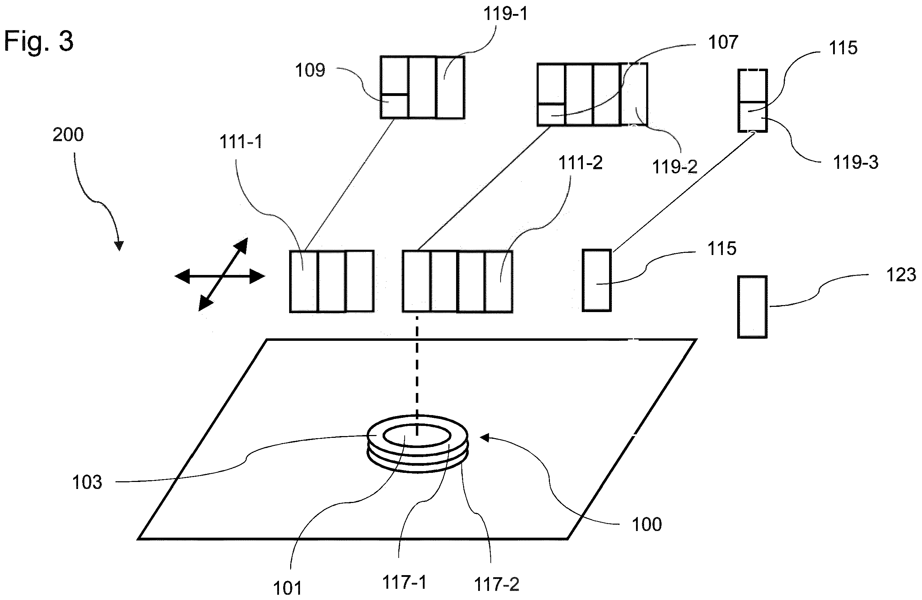

Fig. 3 zeigt eine schematische Darstellung eines Herstellungsgeräts 200 zum Herstellen einer dentalen Restauration 100 durch Strahldruck (Inkjet-Druck) von wässrigen oder lösungsmittel-basierten Schlickern 109. Um keramische, dentale Multimaterial- und Multicolor-Restaurationen 100 mittels Strahldruck additiv zu fertigen, werden Basis-Schlicker 109 verarbeitet.

Das Herstellungsgerät 200 umfasst eine Mehrzahl von Aufnahmebehältern 119-1, in denen die Basis-Schlicker 109 mit unterschiedlichen optischen Eigenschaften angeordnet sind. Zudem umfasst das Herstellungsgerät 200 zumindest mehrere Aufnahmebehälter 119-2, in denen die Färbelösungen 107 aufgenommen sind. Zusätzlich umfasst das Herstellungsgerät 200 einen Aufnahmebehälter 119-3, in dem eine Fixierlösung 115 aufgenommen ist.

Die keramischen Schlicker 109 werden jeweils mittels eines zugeordneten Druckkopfes 111-1 tröpfchenweise in mehreren Schichten 117-1, ..., 117-n aufgebracht, um die dentale Restauration 100 schichtweise räumlich aufzubauen. Der Druckkopf 111-1 ist in zwei Richtungen beweglich, so dass der Schlicker 109 an jeder Position aufgedruckt werden kann. Der oder die Druckköpfe können auch fest angeordnet sein und die Bauplattform bewegt sich in den drei Raumrichtungen unterhalb der Druckköpfe.

Für den selektiven Materialauftrag werden Schlicker 109 mit einem Tropfenvolumen von typischerweise 10 bis 100 pL verwendet, durch deren Einsatz der zeitlich aufwändige Entbinderungsprozess entfällt. Zum Ausstoßen der Tropfen werden beispielsweise elektrisch gesteuerte Piezoelemente verwendet.

Für die Auftragung der Färbelösungen 107 wird ein weiterer Druckkopf 111-2 verwendet, durch den die Färbelösungen 107 auf eine oder mehrere Schichten 117-1, ..., 117-n strahlgedruckt werden können. Der Druckkopf 111-2 ist ebenfalls in zwei Richtungen beweglich, so dass die Färbelösungen 107 an jeder Position aufgedruckt werden können. Die Druckköpfe 111-1 und 111-2 können unabhängig voneinander steuerbar sein oder in einem gemeinsamen Druckmodul integriert sein. Die Druckköpfe 111-1 und 111-2 können auch fest angeordnet sein und die Bauplattform bewegt sich in den drei Raumrichtungen unterhalb der Druckköpfe.

Die Fixierlösung 115 wird durch eine weitere Auftragungsvorrichtung 121 aufgetragen, die durch einen Sprühkopf zum flächigen Auftragen der Fixierlösung 115 oder einen weiteren Druckkopf zum selektiven Auftragen der Fixierlösung 115 gebildet sein kann.

Zum Trocknen des wässrigen Materials ist eine Trocknungsvorrichtung 123 vorgesehen, mit der die Schichten 117-1, ..., 117-n, die Färbelösung 107 und die Fixierlösung 115 rissfrei getrocknet werden können. Die Trocknungsvorrichtung 123 ist beispielsweise durch ein Gebläse und/oder einen Infrarotstrahler gebildet.

Das Herstellungsgerät 200 stellt eine verringerte Anzahl an voreingefärbten und Yttrium-dotierten neutralen Basis-Schlickern 109 bereit, um die Anzahl der Druckköpfe 111-1 und 111-2 zu minimieren und dennoch ein ästhetisches und funktionelles Ergebnis der dentalen Restauration 100 zu erzielen. Die Farbgebung erfolgt getrennt durch das selektive Aufbringen von Färbelösungen 107.

Die gewünschte Zahnfarbe wird aus den bereits voreingefärbten Färbelösungen 107 zusammengesetzt und gemischt. Dabei wird ein subtraktives Farbsystem verwendet, das einen eingeschränkten, dentalen Farbraum (Dental Color Gamut) aufspannt. Die Farbmischung, das dreidimensionale Halftoning oder Dithering, dieser voreingefärbten Schlicker 109 in verschiedenen Verhältnissen entsteht dann in dem eingeschränkten, dentalen Farbraum (Gamut), der die gängigen Zahnfarben, jedoch nicht alle Farben abdeckt.

Dabei werden unterschiedliche Färbelösungen 107 über einen 3D-Dithering-Algorithmus auf eine Schicht 117-1, ..., 117-n selektiv aufgetragen, der durch ein Dithering-Modul 113 ausgeführt wird. Das Dithering-Modul 113 umfasst hierzu einen Prozessor zum Ausführen des 3D-Dithering-Algorithmus und einen digitalen Datenspeicher zum Speichern der berechneten Mischungsverhältnisse und des Dithering-Algorithmus. Der Prozessor umfasst jedes Hardwaresystem, jede Komponente oder jeden Mechanismus, der Daten, Signale oder andere Informationen verarbeitet. Ein Prozessor kann ein System mit einer zentralen Datenverarbeitungseinheit (CPU), mehreren Datenverarbeitungseinheiten (MPU), eine dedizierte elektrische Schaltung zum Erreichen der Funktionalität oder andere Systeme umfassen. Der Datenspeicher kann Festplatten, Flash-Speicherkarten, wahlfreie Zugriffsspeicher (RAM) oder Nur-Lesespeicher (ROM) umfassen.

Bei dem Dithering werden die unterschiedlichen Färbelösungen in einem bestimmten Verhältnis und zweidimensionalen Druckmuster in der Druckebene selektiv mittels des Strahldruckverfahrens aufgetragen. Der 3D-Dithering-Algorithmus berechnet zudem, dass nicht gleiche zweidimensionale Dithering-Muster in mehreren Schichten 117-1, ..., 117-n übereinander aufgetragen werden. Dadurch können bei senkrechten Flächen optische Artefakte wie Streifen- oder Moiré-Muster verhindert werden. Die Farbmischung, das dreidimensionale Halftoning oder Dithering dieser voreingefärbten Schlicker 109 in verschiedenen Verhältnissen entsteht innerhalb des eingeschränkten, dentalen Farbraums (Gamut), der die gängigen Zahnfarben, jedoch nicht alle allgemeinen Farben abdeckt.

Bei dem Einsatz von hochviskosen Schlickern 109 mit einer Viskosität von mehr als 100 mPas mit einem hohen Füllgrad werden besondere Anforderungen an den Druckkopf 111-1 und sein Fluidsystem gestellt. Der Druckkopf 111-1 ist mit dem verwendeten Binder und/oder dem Trägermaterial des Schlickers 109 kompatibel, um Unverträglichkeiten und Folgen wie z.B. Korrosion am Druckkopf zu vermeiden.

Nach dem selektiven Materialauftrag einer Schicht 117-1, ..., 117-n des Schlickers 109 mittels des Strahldruckverfahrens wird diese Schicht 117-1, ..., 117-n rissfrei getrocknet, indem das Wasser oder das Lösungsmittel als Binder verdampft wird. Dabei kann das Keramikpulver des Schlickers 109 bereits in einer Farbe eingefärbt sein, beispielsweise einer Grundfarbe oder Zahnfarbe. Zurück bleibt eine poröse Weißling-Schicht, die eine Schichtdicke von 1 um bis 50 um und eine Dichte von xx% aufweist. Dieser Prozess wird wiederholt, bis die gesamte dentale Restauration 100 schichtweise räumlich aufgebaut ist.

Stehen zwei Schlicker 109 mit unterschiedlichen Transluzenzen zur Verfügung, kann der hochfeste und opake Schlicker 109 für den Dentinkern und der normalfeste und hochtransluzente Schlicker 109 für die Schneide eingesetzt werden. Die selektive Einfärbung erfolgt jeweils nach 1-n Schichten 117-1, ..., 117-n des Materialauftrags durch das selektive Strahldrucken von Färbelösungen, wie beispielsweise NitratLösungen (Säuren), nach einer entsprechenden Farbkodierung oder Farbinformation.

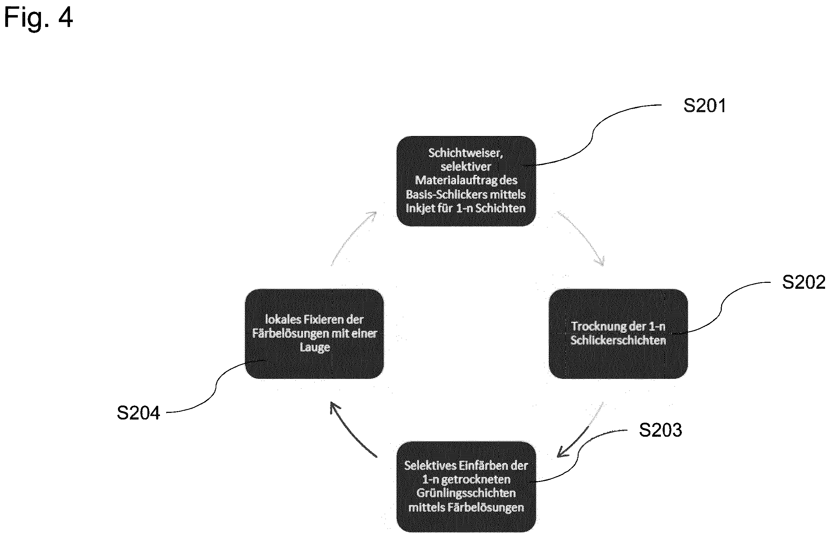

Fig. 4 zeigt eine schematische Ansicht eines Verfahrens zum Herstellen der dentalen Restauration 100 durch Strahldruck. Im Schritt S201 erfolgt zunächst ein schichtweiser selektiver Materialauftrag eines Schlickers 109 mittels eines Strahldruckverfahrens (Inkjet-Verfahren) für 1-n Schichten 117-1, ..., 117-n.

In Schritt S202 wird die aufgebrachte Schlickerschicht getrocknet, beispielsweise durch Zuführen von Warmluft. In Schritt S203 werden die 1 bis n aufgebrachten Schichten 117-1, ..., 117-n des Schlickers 109 (Grünlingsschichten) mittels Färbelösungen selektiv eingefärbt. Hierbei werden die Färbelösungen ebenfalls mittels Strahldruck auf die 1 bis n aufgebrachten Schichten 117-1, ..., 117-n aufgedruckt. In Schritt S204 werden die aufgedruckten Färbelösungen mittels einer Fixierlösung lokal fixiert, d.h. mittels einer alkalischen Lösung mit einem pH-Wert, der größer als 7 ist (Lauge). Anschließend werden die Schritte S201 bis S204 so lange wiederholt, bis die dentale Restauration 100 vollständig räumlich aufgebaut ist.

Für das Verfahren können entweder wässrige oder lösungsmittel-basierte Schlicker 109 verarbeitet werden. Ein selektiver Materialauftrag (Schritt S201) und eine selektive Einfärbung (Schritt S203) erfolgen dabei getrennt. Der Prozess hat den technischen Vorteil, dass bei getrockneten, d.h. wasserfreien Schichten 117-1, ..., 117-n der pH-Wert der Färbelösung kaum eine Rolle spielt.

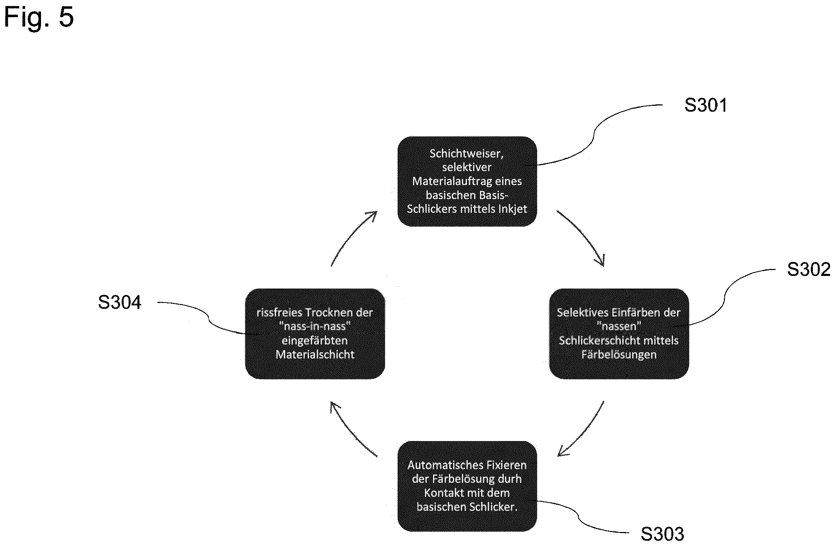

Fig. 5 zeigt eine schematische Ansicht eines weiteren Verfahrens zum Herstellen der dentalen Restauration 100 durch Strahldruck. Im Schritt S301 erfolgt zunächst ein schichtweiser selektiver Materialauftrag eines basischen Schlickers 109 mittels eines Strahldruckverfahrens (Inkjet-Verfahren) für 1-n Schichten.

Der basische Schlicker 109 weist einen pH-Wert von größer als 7 auf. Ein üblicher basischer Schlicker 109 enthält H2O, Dispergierhilfsmittel, wie beispielsweise 0 bis 5 Gew.-%, bevorzugt 0.01 bis 5 Gew.% Carbonsäurederivate (Zitronensäure) oder 0 bis 5 Gew.-% Ammoniumpolyacrylsäure (NH4PAA), bevorzugt 0.01 bis 5 Gew.%. Der pH-Wert (8-11) kann mit NH4OH eingestellt werden. Die basische Einstellung des Schlickers 109 hat den Vorteil, dass sich der keramische Schlicker 109 weniger aggressiv in Bezug auf Korrosion gegenüber den Metallteilen des Druckkopfes verhält.

Im Schritt S302 werden die noch nassen und ungetrockneten aufgebrachten Schichten 117-1, ..., 117-n des Schlickers 109 (Grünlingsschichten) mittels der Färbelösungen selektiv eingefärbt. Dabei kommt es zu einer Fällungsreaktion, sobald die Nitratsäure (Färbelösung) auf den feuchten Schlicker 109 auftrifft.

Im Schritt S303 werden die Färbelösungen durch den Kontakt mit dem basischen Schlicker 109 automatisch fixiert. In Schritt S304 werden die nass in nass eingefärbten Schichten 117-1, ..., 117-n rissfrei getrocknet, beispielsweise durch Zuführen von Warmluft. Anschließend werden die Schritte S301 bis S304 so lange wiederholt, bis die dentale Restauration 100 vollständig räumlich aufgebaut ist.

Für das Verfahren können entweder wässrige oder lösungsmittel-basierte Schlicker 109 verarbeitet werden. Ein selektiver Materialauftrag (Schritt S301) und eine selektive Einfärbung (Schritt S302) erfolgen dabei getrennt.

Falls kein basischer Schlicker 109 verwendet wird, diffundiert die Färbelösung 107 in die Schichten 117-1, ..., 117-n. Die Fixierung der aufgetragenen Färbelösung erfolgt durch separaten Auftrag der Fixierlösung, wie beispielsweise einer Lauge.

Bei dem Prozess wird der Schlicker 109 im sauren pH-Wert Bereich stabilisiert. Nach dem Drucken und Trocknen der jeweiligen Einzelschichten wird zuerst eine basische Fixierlösung aufgebracht, wie z.B. 0.1N AmmoniumhydroxidLösung. Nachfolgend wird die Färbelösung 107 aufgebracht. Beim Auftreffen der Färbelösung auf die mit Fixierlösung behandelte Schicht wird der pH-Wert der Färbelösung örtlich in Richtung pH>2 verschoben. Mit dem Verschieben des pH-Werts der Färbelösung entstehen örtlich an Stellen, an denen die Färbelösung fixiert werden soll, Metallhydroxide, wie beispielsweise Fe(OH)3. Dieser Prozess wird als Fällung oder Ausflocken bezeichnet.

Nach dem Drucken und Trocknen der jeweiligen Einzelschichten kann auch die Färbelösung und unmittelbar danach die basische Fixierlösung aufgebracht werden, die wiederum die färbenden Komponenten an der gewünschten Position fixiert.

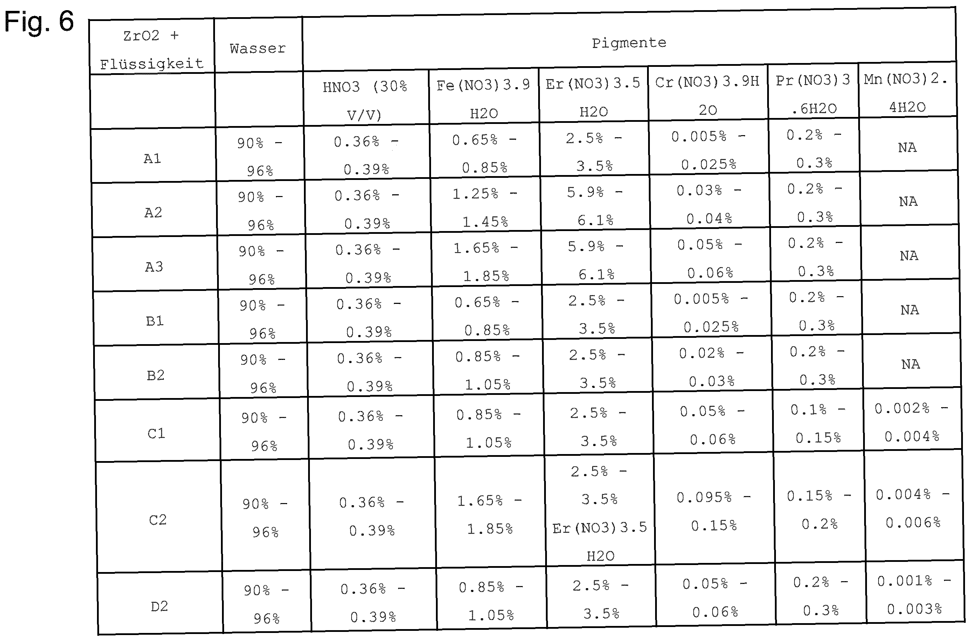

Fig. 6 zeigt eine Tabelle mit den Zusammensetzungen unterschiedlicher Färbelösungen 107. Die Färbelösungen 107 sind niederviskos und können entweder mittels eines Tintenstrahl-Druckkopfes mit DOD-Technologie oder Bubble-Jet-Technologie strahlgedruckt werden. In diesem Druckkopf können für einen Multifarbdruck mehrere Farbkanäle integriert sein. Der Druckkopf kann die Färbelösung in einer hohen Auflösung von 720 dpi auf die Schicht 117-1, ..., 117-n aufbringen.

Bei sechs verfügbaren Druckköpfen wird nur ein einziger Druckkopf für Schlicker 109 mittlerer Transluzenz und ein Druckkopf für Stützmaterial verwendet. Bei sieben verfügbaren Druckköpfen werden zwei Druckköpfe für jeweils einen opaken Schlicker 109 und einen hochtransluzenten Schlicker 109 und ein Druckkopf für Stützmaterial verwendet. Die jeweils anderen vier Druckköpfe werden für die Färbelösungen 107 verwendet.

Die Färbelösungen sind Nitratsalz oder Chloridsalzlösungen auf wässriger Basis. Dazu werden verschiedene Metallsalze (z.B. Fe(NO3)3*9 H2O; Pr(NO3)3*6 H2O; Tb(NO3)3*5 H2O; Er(NO3)3*5 H2O; Mn(NO3)2*4 H2O; Co(NO3)2*6 H2O; Cr(NO3)3*9 H2O; Mg(NO3)2*6 H2O; Al(NO3)3*9 H2O; Cu(NO3)2*3 H2O; Zn(NO3)2*6 H2O; Y(NO3)3*6 H2O; La(NO3)3*6 H2O; Ce(NO3)3*6 H2O; Nd(NO3)3*6 H2O; Sm(NO3)3*6 H2O; Gd(NO3)3*6 H2O; Yb(NO3)3*6 H2O); Ni(NO3)2* 6H2O; Co(NO3)2 *6H2O; Ga(NO3)3* xH2O; In(NO3)3 xH2O, in unterschiedlichen Konzentrationen gelöst.

Die Färbelösungen 107 können aber je nach gewünschter Zusammensetzung auch deutlich höher konzentriert sein. Durch eine Steigerung der Ionen-Konzentration steigt aber eine Viskosität an oder der pH-Wert verschiebt sich in den sauren Bereich (<7).

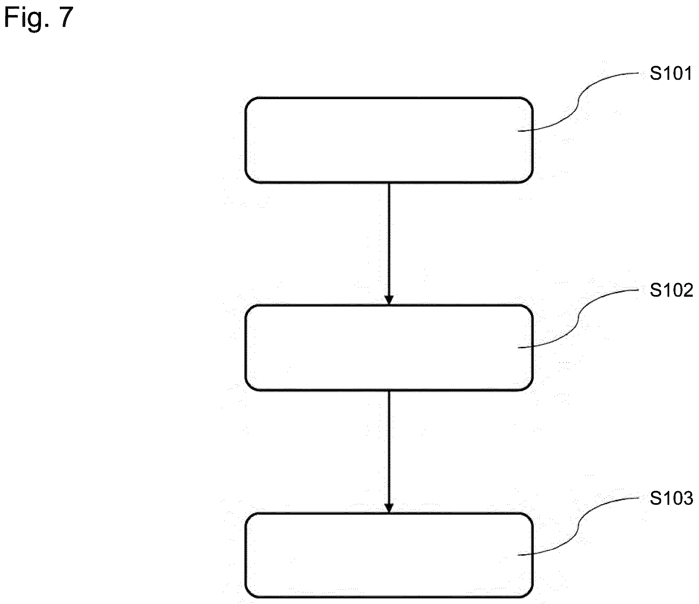

Fig. 7 zeigt ein Blockdiagramm eines Verfahrens zum Herstellen einer dentalen Restauration 100 durch Strahldruck. In Schritt S101 werden die Schichten 117-1, ..., 117-n der dentalen Restauration 100 mittels des keramischen Schlickers 109 strahlgedruckt. In Schritt S102 wird die Färbelösung 107 auf die eine oder mehreren Schichten 117-1, ..., 117-n strahlgedruckt. Anschließend wird in Schritt S103 die Fixierlösung 115 zum Fixieren der Färbelösung 107 aufgetragen. Diese Schritte werden so lange wiederholt, bis die dentale Restauration 100 vollständig aufgebaut ist.

Wird die Färbelösung 107 in die zuvor getrocknete Schicht 117 1, ..., 117-n strahlgedruckt und danach wieder feuchter Schlicker auf die vorherige, getrocknete und eingefärbte Schicht 117-1, ..., 117-n aufgetragen, kann es passieren, dass die Färbelösung 107 wieder angelöst wird und unkontrolliert in der bereits aufgebauten dentalen Restauration 100 diffundiert. Bei dem Strahldrucken der Färbelösung 107 auf die getrocknete Schicht 117-1, ..., 117-n ist darauf zu achten, dass die Eindringtiefe der Färbelösung 107 kontrollierbar ist.
Ist beispielsweise die Infiltrationstiefe größer als eine Schichtdicke, kann die Einfärbung erst nach multiplem Schichtauftrag des Schlickers 109 erfolgen.

Durch das Auftragen der Fixierlösung 115 wird die Färbelösung 107 lokal fixiert, so dass durch Ausfällen der Hydroxide ein unkontrolliertes Diffundieren oder Vermischen verhindert wird. Dies kann durch das großflächige oder selektive Auftragen einer basischen Fixierlösung 115 erfolgen, wie z.B. einer Ammoniak-Ammoniumchlorid-Pufferlösung, um den pH-Wert zu verschieben. Die Ammoniak-Ammoniumchlorid-Pufferlösung umfasst beispielsweise 987.8 g Wasser, 6.8 g 25%-iges Ammoniak und 5.4 g Ammoniumchlorid.

Es kann ein einziger Schlicker 109 in einer Grundeinfärbung und einer mittleren Transluzenz für den additiven Materialauftrag der dentalen Restauration 100 verwendet werden. Die selektive Einfärbung des Schlickers 109 erfolgt dabei über das Strahldrucken (Jetten) der Färbelösungen, die anschließend fixiert werden.

Es können unterschiedliche Druckkopftechnologien eingesetzt werden, da die Schlicker 109 und die Färbelösung 107 unterschiedliche rheologische Eigenschaften aufweisen. Der Schlicker 109 ist hochviskos (10 - 1000 mPas abhängig von der Scherrate) und beinhaltet einen hohen Gewichts-/Volumenanteil eines abrasiven Füllstoffs in Form von Keramikpartikeln. Vorteilhaft ist eine niedrige Viskosität des Schlickers 109 zwischen 10 - 500 mPas und noch vorteilhafter ist eine Viskosität zwischen 10 - 100 mPas. Je höher die Viskosität des Schlickers 109 ist, desto schwieriger ist die Verarbeitung mittels Inkjet-Druckköpfen. Außerdem sollte der Druckkopf in der Lage sein, wässrige Medien zu jetten.

Die Färbelösung 107 ist dagegen niedrigviskos (0.5 - 50 mPas). Da es sich hier um hochkonzentrierte Nitratlösungen handelt, sollte der verwendete Druckkopf resistent gegen Nitrate sein. Zudem sind die jeweils verarbeiteten Volumen unterschiedlich und liegen in einem Mengenverhältnis von ca. 98% für den Schlicker 109 und bei 2% für die Färbelösung 107. Das selektive Aufbringen von Färbelösungen 107 kann außerdem über einen Mehrkanal-Druckkopf 111-2 erfolgen.

Die Eindring- oder Diffusionstiefe der Färbelösung 107 kann außerdem über die Einstellung der Viskosität gesteuert werden. Dies erfolgt durch Zusatz eines geeigneten Verdickungsmittels zur Färbelösung 107, das im pH-Bereich der Färbelösung stabil ist, wie beispielsweise Polyvinylpyrolidon (PVP).

Das Hauptvolumen wird über den Schlicker 109 verarbeitet, wohingegen von der Färbelösung 107 weniger Volumen verarbeitet wird. Die Färbelösung 107 und der Schlicker 109 können in unterschiedlich großen Aufnahmebehältern 119-1 und 119-2 gespeichert werden.

Danach wird die vollständig aufgebaute dentale Restauration 100 in einem Sinterofen gesintert. Die Schlicker 109 und die Färbelösungen 107 können auf ein einheitliches Sinterverhalten abgestimmt sein. Die Sinterkinetik kann durch eine Anpassung der einzelnen Färbelösungen 107 homogen eingestellt werden. Die Sinter-Aktivatoren werden durch den Einsatz oder die Zugabe von Sinterinhibitoren "neutralisiert".

Für den selektiven Einfärbeprozess können Nitratlösungen als Färbelösung 107 verwendet werden, unabhängig davon, ob die Schlicker 109 bereits voreingefärbt sind oder die Einfärbung ausschließlich über den schichtweise selektiven Auftrag der Färbelösungen 107 während des additiven, schichtweisen Fertigungsprozesses erfolgt.

Zusätzlich kann durch variable Bestandteile von Yttrium oder Ytterbium in der Färbeflüssigkeit die Opazität des Schlickers 109 der dentalen Restauration 100 eingestellt werden. So werden beispielsweise die Elemente Yttrium, Ytterbium, Neodym und Europium als Transluzenz-Verstärker (Translucency Enhancer) und Aluminium und Silizium als opakes Liquid eingesetzt. Mit einer geeigneten Färbelösung kann der Yttrium-Gehalt erhöht werden. Somit können unterschiedliche Opazitätswerte in der dentalen Restauration 100 erreicht werden, wie beispielsweise opak für den Dentinkern und transluzent für die Schneide.

### BEZUGSZEICHENLISTE

- 100: Dentale Restauration
- 101: Dentinkern
- 103: Zahnschmelz
- 105: Zahn
- 107: Färbelösung
- 109: Schlicker
- 111: Druckkopf
- 113: Dithering-Modul
- 115: Fixierlösung
- 117: Schicht
- 119: Aufnahmebehälter
- 121: Auftragungsvorrichtung
- 123: Trocknungsvorrichtung

## Patentansprüche

1. Verfahren zum Herstellen einer dentalen Restauration (100) durch Strahldruck, mit den Schritten:
- Strahldrucken (S101) einer oder mehrerer Schichten (117-1, ..., 117-n) der dentalen Restauration (100) mittels eines keramischen Schlickers (109);
- Strahldrucken (S102) einer Färbelösung (107) auf die eine oder mehreren Schichten (117-1, ..., 117-n);
**gekennzeichnet durch**
- Auftragen (S103) einer Fixierlösung (115) zum Fixieren der Färbelösung (107).

2. Verfahren nach Anspruch 1, wobei die Fixierlösung (115) auf die eine oder mehreren Schichten (117-1, ..., 117-n) gesprüht wird.

3. Verfahren nach Anspruch 1, wobei die Fixierlösung (115) auf die eine oder mehreren Schichten (117-1, ..., 117-n) mittels eines Druckkopfes (111) strahlgedruckt wird.

4. Verfahren nach einem der vorangehenden Ansprüche, wobei die eine oder mehreren Schichten (117-1, ..., 117-n) vor oder nach dem Strahldrucken der Färbelösung (107) getrocknet werden.

5. Verfahren nach einem der vorangehenden Ansprüche, wobei die eine oder mehreren Schichten (117-1, ..., 117-n) vor oder nach dem Auftragen der Fixierlösung (115) getrocknet werden.

6. Verfahren nach einem der vorangehenden Ansprüche, wobei ein pH-Wert der Fixierlösung (115) größer als 7 ist.

7. Verfahren nach einem der vorangehenden Ansprüche, wobei die Fixierlösung (115) eine Ammoniak-AmmoniumchloridLösung ist.

8. Verfahren nach einem der vorangehenden Ansprüche, wobei die Schritte (S101, ..., S103) zum Herstellen der dentalen Restauration (100) wiederholt werden.

9. Verfahren nach einem der vorangehenden Ansprüche, wobei die dentale Restauration (100) in einem Sinterofen gesintert wird.

10. Herstellungsgerät (200) zum Herstellen einer dentalen Restauration (100) durch Strahldruck, mit:
- einem ersten Druckkopf (111-1) zum Strahldrucken einer oder mehrerer Schichten (117-1, ..., 117-n) der dentalen Restauration (100) mittels eines keramischen Schlickers (109); und
- einem zweiten Druckkopf (111-2) zum Strahldrucken einer Färbelösung (107) auf die eine oder mehreren Schichten (117-1, ..., 117 n); und
- einer Auftragungsvorrichtung (121) zum Auftragen einer Fixierlösung (115) zum Fixieren der Färbelösung (107);
**gekennzeichnet durch**
- einen Aufnahmebehälter (119-3), in dem eine Fixierlösung (115) aufgenommen ist.

11. Herstellungsgerät (200) nach Anspruch 10, wobei die Auftragungsvorrichtung (121) durch einen Sprühkopf zum flächigen Auftragen der Fixierlösung (115) gebildet ist.

12. Herstellungsgerät (200) nach Anspruch 10, wobei die Auftragungsvorrichtung (121) durch einen Druckkopf (111) zum selektiven Auftragen der Fixierlösung (115) gebildet ist.

13. Herstellungsgerät (200) nach einem der Ansprüche 10 bis 12, wobei das Herstellungsgerät (200) eine Trocknungsvorrichtung (123) zum Trocknen der aufgetragenen Materialien umfasst.

14. Herstellungsgerät (200) nach Anspruch 13, wobei die Trocknungsvorrichtung (123) ein Gebläse und/oder einen Infrarotstrahler umfasst.

## Claims

1. A method of producing a dental restoration (100) by jet-printing, comprising:
- jet-printing (S101) one or more layers (117-1, ..., 117-n) of the dental restoration (100) using a ceramic slurry (109);
- jet-printing (S102) a coloring solution (107) onto the one or more layers (117-1, ..., 117-n);
**characterized by**
- applying (S103) a fixing solution (115) for fixing the coloring solution (107).

2. The method according to claim 1, wherein the fixing solution (115) is sprayed onto the one or more layers (117-1, ..., 117-n).

3. The method according to claim 1, wherein the fixing solution (115) is jet-printed onto the one or more layers (117-1, ..., 117-n) by a print head (111).

4. The method according to any one of the preceding claims, wherein the one or more layers (117-1, ..., 117-n) are dried before or after jet-printing the coloring solution (107).

5. The method according to any one of the preceding claims, wherein the one or more layers (117-1, ..., 117-n) are dried before or after applying the fixing solution (115).

6. The method according to any one of the preceding claims, wherein a pH of the fixing solution (115) is greater than 7.

7. The method according to any one of the preceding claims, wherein the fixing solution (115) is an ammonia-ammonium chloride solution.

8. The method according to any one of the preceding claims, wherein the steps (S101, ..., S103) to produce the dental restoration (100) are repeated.

9. The method according to any one of the preceding claims, wherein the dental restoration (100) is sintered in a sintering furnace.

10. A production apparatus (200) for producing a dental restoration (100) by jet-printing, comprising:
- a first print head (111-1) for jet-printing one or more layers (117-1, ..., 117-n) of the dental restoration (100) by means of a ceramic slurry (109); and
- a second print head (111-2) for jet-printing a coloring solution (107) onto the one or more layers (117-1, ..., 117-n); and
- an application device (121) for applying a fixing solution (115) for fixing the coloring solution (107);
**characterized by**
- a storage container (119-3) which receives a fixing solution (115).

11. The production apparatus (200) according to claim 10, wherein the application device (121) is formed by a spray head for applying the fixing solution (115) extensively.

12. The production apparatus (200) according to claim 10, wherein the application device (121) is formed by a print head (111) for selectively applying the fixing solution (115).

13. The production apparatus (200) according to any one of claims 10 to 12, wherein the production apparatus (200) comprises a drying device (123) for drying the applied materials.

14. The production apparatus (200) according to claim 13, wherein the drying device (123) comprises a blower and/or an infrared radiator.

## Revendications

1. Procédé de fabrication d'une restauration dentaire (100) par impression par jet, comprenant les étapes suivantes :
- l'impression par jet (S101) d'une ou plusieurs couches (117-1, ..., 117-n) de la restauration dentaire (100) au moyen d'une barbotine céramique (109);
- l'impression par jet (S102) d'une solution colorante (107) sur la ou les couches (117-1, ..., 117-n);
**caractérisé par**
- l'application (S103) d'une solution de fixation (115) pour fixer la solution colorante (107).

2. Procédé selon la revendication 1, où la solution de fixation (115) est pulvérisée sur la ou les couches (117-1, ..., 117-n).

3. Procédé selon la revendication 1, où la solution de fixation (115) est imprimée par jet sur la ou les couches (117-1, ..., 117-n) au moyen d'une tête d'impression (111).

4. Procédé selon l'une des revendications précédentes, où la ou les couches (117-1, ..., 117-n) sont séchées avant ou après l'impression par jet de la solution colorante (107).

5. Procédé selon l'une des revendications précédentes, où la ou les couches (117-1, ..., 117-n) sont séchées avant ou après l'application de la solution de fixation (115).

6. Procédé selon l'une des revendications précédentes, où une valeur de pH de la solution de fixation (115) est supérieure à 7.

7. Procédé selon l'une des revendications précédentes, où la solution de fixation (115) est une solution d'ammoniac et de chlorure d'ammonium.

8. Procédé selon l'une des revendications précédentes, où les étapes (S101, ..., S103) sont répétées pour fabriquer la restauration dentaire (100).

9. Procédé selon l'une des revendications précédentes, où la restauration dentaire (100) est frittée dans un four de frittage.

10. Dispositif de fabrication (200) pour fabriquer une restauration dentaire (100) par impression par jet, comprenant :
- une première tête d'impression (111-1) destinée à imprimer par jet une ou plusieurs couches (117-1, ..., 117-n) de la restauration dentaire (100) à l'aide d'une barbotine céramique (109); et
- une deuxième tête d'impression (111-2) destinée à imprimer par jet une solution colorante (107) sur la ou les couches (117-1, ..., 117 n); et
- un dispositif d'application (121) pour appliquer une solution de fixation (115) destinée à fixer la solution colorante (107);
**caractérisé par**
- un récipient (119-3) contenant une solution de fixation (115).

11. Dispositif de fabrication (200) selon la revendication 10, où le dispositif d'application (121) est formé par une tête de pulvérisation destinée à appliquer la solution de fixation (115) sur toute la surface.

12. Dispositif de fabrication (200) selon la revendication 10, où le dispositif d'application (121) est formé par une tête d'impression (111) destinée à appliquer de manière sélective la solution de fixation (115).

13. Dispositif de fabrication (200) selon l'une des revendications 10 à 12, où le dispositif de fabrication (200) comprend un dispositif de séchage (123) destiné à sécher les matériaux appliqués.

14. Dispositif de fabrication (200) selon la revendication 13, où le dispositif de séchage (123) comprend un ventilateur et/ou un émetteur infrarouge.
